# EUROPÄISCHE PATENTSCHRIFT

(11) **EP 1 216 664 B1**
(45) Veröffentlichungstag und Bekanntmachung des Hinweises auf die Patenterteilung: **01.12.2010**
(21) Anmeldenummer: 01125206.1
(22) Anmeldetag: 24.10.2001
(51) Int. Cl.: A61B 17/54

(54) **Vorrichtung zur abtragenden Behandlung von Hornhaut**
Device for abrasive treatment of calloused skin
Dispositif pour le traitement abrasif des durillons

(30) Priorität: 16.11.2000 DE 20019442 U
(43) Veröffentlichungstag der Anmeldung: 26.06.2002
(73) Patentinhaber: CREDO STAHLWARENFABRIK GUSTAV KRACHT GMBH & CO. KG, 42653 Solingen (DE)
(72) Erfinder: Dembski, Reinhard, 42699 Solingen (DE)
(74) Vertreter: Stenger, Watzke & Ring

(56) Entgegenhaltungen:
- DE-A- 3 320 594
- DE-C- 363 214
- DE-C- 512 593
- DE-U- 29 919 293
- US-A- 3 636 625
- US-A- 3 797 505

## Beschreibung

Die Erfindung betrifft eine Vorrichtung zur abtragenden Behandlung von Hornhaut, mit einer Handhabe, einem sich an die Handhabe anschließenden Befestigungsbereich und einem Aufsatz, der eine Baugruppe bestehend aus einem Halteelement und einer Klinge ist, wobei das Halteelement im Befestigungsbereich kraftschlüssig festlegbar ist.

Es sind Vorrichtungen in unterschiedlichsten Ausführungen bekannt, die in der Körperpflege zum Abtragen von verhornten Zellschichten in Hautbereichen eingesetzt werden. Unter anderem werden derartige Vorrichtungen auch zum Abtragen von Hühneraugen verwendet. Die verwendeten Vorrichtungen sind zu unterscheiden in Hornhautraspeln mit einer Vielzahl von Zähnen, und in Hornhauthobeln mit ein- oder mehrschneidigen Klingen. Um eine einfache Reinigung zu ermöglichen und um ein verschlissenes Funktionselement ersetzen zu können, können diese Funktionselemente austauschbar in der Vorrichtung angeordnet sein.

Aus dem deutschen Gebrauchsmuster 299 01 517 ist ein Hornhauthobel bekannt, bei dem sich die Klinge austauschbar einspannen läßt. Dazu ist die Klinge zwischen einem vorderen Einspannabschnitt des Handgriffs und einem entsprechend ausgebildeten Befestigungselement einspannbar. Das Befestigungselement ist in einer ersten Stellung um eine senkrecht zur Längsrichtung des Handgriffs verlaufende Schwenkachse verschwenkbar und in einer zweiten Stellung durch an dem Einspannabschnitt ausgebildete Führungen relativ zu dem Handgriff verschiebbar. Durch Verschiebung in Richtung auf einen hinteren Handhabungsabschnitt des Handgriffs ist das Befestigungselement kraftschlüssig an dem Einspannabschnitt festlegbar. Dazu ist die offene Klinge manuell auf das Befestigungselement aufzulegen, wobei eine gewisse Verletzungsgefahr besteht.

Eine gattungsgemäße Vorrichtung zur abtragenden Behandlung von Hornhaut ist beispielsweise aus der US 3,636,62 bekannt geworden. Der Oberbegriff des Anspruchs 1 basiert auf dieser Offenbarung. Die Besonderheit der hier beschriebenen Vorrichtung besteht darin, daß die Klinge auch um 90° verdreht ausgerichtet werden kann, was ein Schälen von Hornhaut ermöglicht. Ein weiteres Dokument, das eine gattungsgemäße Vorrichtung betrifft, ist die US 3,797,505. Der hier beschriebene Hornhauthobel verfügt zur Festlegung der Klinge über ein Halteelement, das am Hobelkopf im endmontierten Zustand in parallel ausgerichteten Nuten einrastet. Auf diese Weise erfolgt eine Festlegung der Klinge gegenüber dem Hobelkopf.

Der Erfindung liegt daher die **Aufgabe** zugrunde, die Vorrichtung zur abtragenden Behandlung von Hornhaut mit einer Handhabe und einem an die Handhabe anschließenden Befestigungsbereich für ein Funktionselement in Form einer Klinge derart weiterzubilden, daß eine weitgehend sichere Handhabung des Klingenwechsels erreichbar ist.

Bei der Vorrichtung der oben genannten Art wird diese Aufgabe dadurch **gelöst**, daß die Klinge durch das Halteelement und innerhalb diesem während ihrer gesamten Handhabung derart gehalten ist, daß ein Benutzer ein Austauschen der Klinge durch ein Austauschen des Halteelements ausführen kann, wobei das Halteelement Griffbereiche bietet, an denen ein Benutzer zum Austausch des Aufsatzes den Aufsatz greifen kann, und wobei der Aufsatz derart ausgebildet ist, daß eine Klinge mit zwei gegenüberliegend angeordneten Schneiden verwendbar ist, so daß bei Verschleiß eine Schneide der Klinge durch umgekehrtes Aufsetzen des Aufsatzes die zweite Schneide der Klinge einsetzbar ist.

In dem Halteelement ist die Klinge während ihrer gesamten Handhabung gehalten, so daß der Benutzer das Austauschen der Klinge durch Austauschen des Halteelements ausführen kann. Eine Handhabung der Klinge selbst kann hierbei vermieden werden. Ferner kann das Halteelement Griffränder aufweisen, an denen ein Benutzer den Aufsatz sicher festhalten und handhaben kann. Dadurch können Verletzungen aufgrund freiliegender scharfer Schneiden und Kanten der Klinge vermieden werden.

Es wird weiter vorgeschlagen, daß der Träger zumindest eine als Befestigungselement für das Halteelement ausgebildete Lasche aufweist. Über Aufschieben des Halteelements gegen die im Träger ausgebildete Lasche kann vorteilhaft eine kraftschlüssige Verbindung zwischen dem Halteelement und der Vorrichtung hergestellt werden.

Vorteilhaft weist die Lasche an ihrer freien Stirnseite eine Abschrägung auf, durch die ein Einführen der Lasche am Halteelement erleichtert werden kann. Die Lasche kann z. B. eine trapezförmige Gestalt aufweisen, um beispielsweise die erforderliche Schubkraft ergonomisch vorteilhaft zu Beginn des Aufbringens des Halteelements gering zu halten und den Kraftschluß erst gegen Ende der Aufsteckbewegung herzustellen.

Daneben wird vorgeschlagen, daß die Klinge durch zumindest ein im Befestigungsbereich angeordnetes Rastelement fixierbar ist. Durch das Rastelement kann die integrale Einheit auch bei nicht sachgerechter Verwendung oder Ermüdung des Kraftschlusses durch häufiges Wechseln der integralen Einheit sicher am Befestigungsbereich gehalten werden.

Der Aufsatz ist an der Vorrichtung kraftschlüssig festlegbar Der Aufsatz weist ein Halteelement auf, welches eine Fakultativ flächige Klinge hält, wobei die Abmessungen des Halteelements die Abmessungen der Klinge übersteigen. Bei entsprechender Ausgestaltung kann erreicht werden, daß der Benutzer vor Verletzungen durch die Klinge weitgehend geschützt ist. Daneben ist der Aufsatz derart ausgestaltet, daß die unter Vorspannung stehende Klinge mit zwei gegenüber liegend angeordneten Schneiden verwendbar ist, so daß bei Verschleiß einer Schneide der Klinge durch umgekehrtes Aufsetzen des Aufsatzes die zweite intakte Schneide der Klinge als Funktionselement einsetzbar ist.

Weiterhin wird vorgeschlagen, daß das Halteelement in Funktionsrichtung gewölbt ist. Eine derartige Wölbung erzeugt die Vorspannung der Klinge und ermöglicht dem Benutzer, beispielsweise auch in Hautsenken oder an dort vorstehenden Kanten gezielt Hornhaut abzutragen.

Dazu wird ferner vorgeschlagen, daß die Klinge durch das Halteelement in Funktionsrichtung in einem gewölbten, unter Biegespannung stehenden Zustand gehalten ist. Durch die Wölbung der Klinge, verursacht durch das entsprechend gewölbte Halteelement, kann eine ergonomisch günstige Wirkungsweise des integrierten Funktionselements erreicht werden, indem beispielsweise durch Ausgestaltung quer zur Funktionsrichtung die Abtragungstiefe von den Rändern zu einer Mittellinie zunimmt. Der Benutzer kann den Hobel bezüglich seiner Längsachse schwenken, um somit die Wirkungstiefe vorzugeben.

Das Halteelement ist vorteilhaft aus Kunststoff gebildet. Mit bekannten Technologien kann kostengünstig und auch umweltverträglich ein geeignetes Halteelement hergestellt werden. Dazu kann das Halteelement beispielsweise derart ausgestaltet sein, daß bei einem verbrauchten Element die Klinge leicht vom Halteelement zu trennen ist, wobei die so getrennten Werkstoffe einer jeweils geeigneten Weiterverwendung zugeführt werden können.

Um das Halteelement vorteilhaft einfach mit der Lasche befestigen zu können, wird vorgeschlagen, daß das Halteelement quer zur Aufsteckrichtung und senkrecht zur flächigen Erstreckung eine bügelförmige Ausformung mit zumindest einer langlochförmigen Ausnehmung aufweist. Die Ausformung, in welche eine Lasche des Trägers eingreifen kann, kann beispielsweise einstückig mit dem Halteelement ausgeführt sein. Dazu kann sie kostengünstig in einem Arbeitsschritt mit dem Halteelement herstellbar sein.

Daneben wird vorgeschlagen, daß zu beiden Seiten des Halteelements jeweils ein Griffrand angebracht ist. Dieser kann dazu beispielsweise seitlich in Aufsteckrichtung am Halteelement angeordnet sein. Dadurch kann der Benutzer den Aufsatz bei geringer Verletzungsgefahr während des Austauschens sicher halten. Daneben können die Griffränder auch seitlich quer zur Aufsteckrichtung angeordnet sein. Eine Aufsteck- und / oder Abziehkraft kann hierdurch beim Austauschen ergonomisch günstig auf den Aufsatz übertragen werden.

Weitere Vorteile und Merkmale sind der folgenden Beschreibung von Ausführungsbeispielen zu entnehmen. Im wesentlichen gleichbleibende Bauteile sind mit den gleichen Bezugszeichen bezeichnet.

Es zeigen:
- Fig. 1: einen Schnitt einer erfindungsgemäßen Vorrichtung entlang einer Linie I -I in Fig. 3,
- Fig. 2: einen Schnitt der Vorrichtung aus Fig. 1 entlang einer Linie II - II in Fig. 3,
- Fig. 3: eine Draufsicht der Vorrichtung aus Fig. 1,
- Fig. 4: die Vorrichtung aus Fig. 4 ohne einen erfindungsgemäßen Aufsatz und
- Fig. 5: einen Aufsatz für eine erfindungsgemäße Vorrichtung.

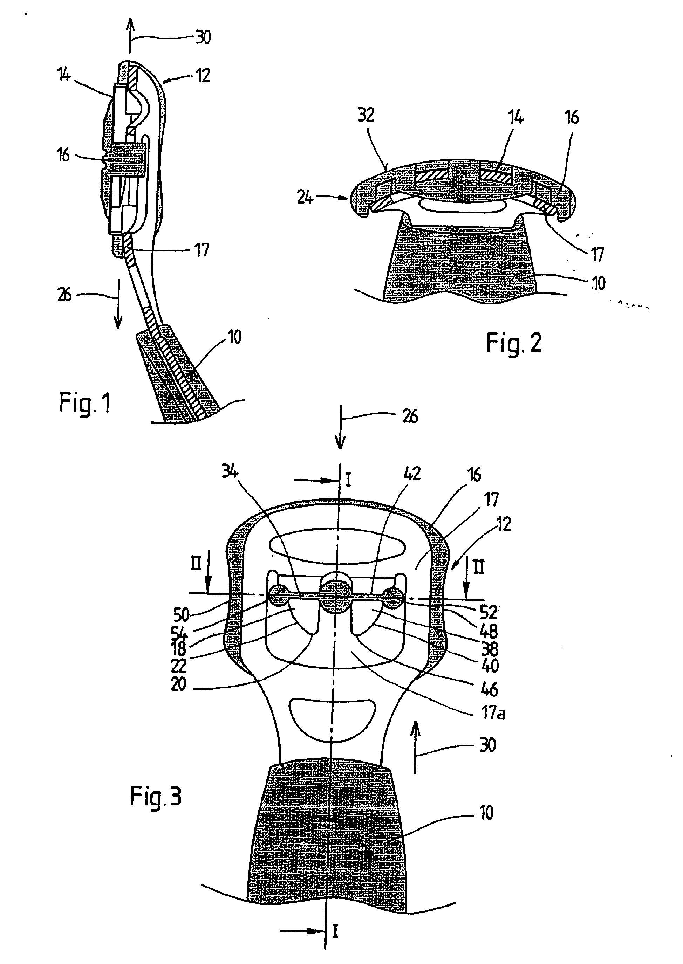

Fig. 1 zeigt einen Schnitt durch eine erfindungsgemäße Vorrichtung zur abtragenden Behandlung von Hornhaut mit einer Handhabe 10 und einem an die Handhabe 10 anschließenden Befestigungsbereich 12 für ein Funktionselement in Form einer Klinge 14. Die Klinge 14 ist rechteckig mit Schneiden an zwei einander abgewandten Rändern des Rechtecks, und sie bildet mit einem Halteelement 16 eine integrale Einheit (Fig. 2), wobei das Halteelement 16 im Befestigungsbereich 12 der Vorrichtung an einem Träger 17 kraftschlüssig festlegbar ist. Der Träger 17 ist entsprechend Fig. 2 gewölbt, verhält sich im wesentlichen starr und ist fest mit der Handhabe 10 verbunden. Der Träger 17 weist eine zentrale Aussparung 17a auf, in die hinein sich zwei als Befestigungselemente für das Halteelement 16 dienende Laschen 18, 38 erstrecken (Fig. 3). Ferner weisen die Laschen 18, 38 an ihren freien Stirnseiten 20, 46 Abschrägungen 22, 40 auf.

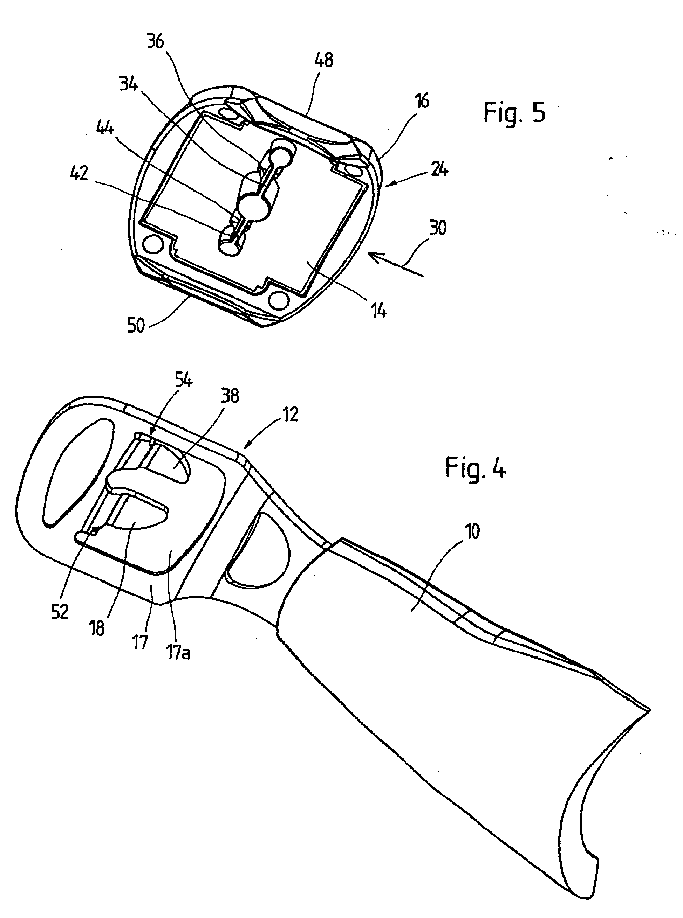

Fig. 5 zeigt einen Aufsatz 24 für die Vorrichtung aus Fig. 1, wobei der Aufsatz 24 an der Vorrichtung kraftschlüssig festlegbar ist. Der Aufsatz 24 ist eine Baugruppe bestehend aus dem Halteelement 16 und der Klinge 14, wobei die Klinge 14 durch das Halteelement 16 und innerhalb diesem gehalten ist. Die Abmessungen des Halteelements 16 übersteigen die Abmessungen der Klinge 14. In Funktionsrichtung 26 ist das Halteelement 16 gemäß Fig. 2 gewölbt. Durch das Halteelement 16 ist auch die Klinge 14 in Funktionsrichtung 26 in einem gewölbten Zustand gehalten. Das Halteelement 16 ist zu diesem Zweck von größerer Festigkeit, als die dünne und daher biegsame Klinge 14.

Quer zu seiner Aufsetzrichtung 30 und senkrecht zu seiner flächigen Erstreckung 32 weist das Halteelement 16 auf der Wölbungsseite zwei stegartige Ausformungen 34, 42 mit Ausnehmungen 36, 44 auf, in die die Laschen 38, 18 kraftschlüssig eingreifen können.

Durch vollständiges Überragen der Klinge 14 durch das Halteelement 16 ist der Benutzer weitgehend gegen Verletzungen geschützt. Zudem bietet das Halteelement 16 zweckmäßige Griffbereiche 48, 50, an denen der Benutzer zum Auswechseln des Aufsatzes 24 den Aufsatz 24 greifen kann, und die zum Aufsetzen oder Abziehen des Aufsatzes 24 erforderliche Kraft ergonomisch günstig übertragen können. Die Griffbereiche 48, 50 sind die Längsränder des Halteelements 16.

Zum Auswechseln des Aufsatzes 24 hält der Benutzer die Vorrichtung an der Handhabe 10 in einer Hand und greift mit der zweiten Hand den Aufsatz 24 an den Griffrändern 48, 50. Bei Einwirkung einer die Haltekraft übersteigenden Kraft in Funktionsrichtung 26 bewegt sich der Aufsatz 24 aus der kraftschlüssigen Verbindung, wobei die Laschen 18, 38 aus den Ausnehmungen 36, 44 herausgleiten. Nachdem die Laschen 18, 38 die Ausnehmungen 36, 44 verlassen haben, kann der Aufsatz 24 mit der darin enthaltenen Klinge ganz abgenommen werden.

Zum Aufstecken eines neuen Aufsatzes 24 wird dieser am Befestigungsbereich 12 derart angeordnet, daß die Laschen 18, 38 vor den Ausnehmungen 36, 44 des Halteelementes 16 liegen. Der Aufsatz 24 wird nun in die Aufsteckrichtung 30 geschoben, wobei die Laschen 18, 38 in die Ausnehmungen 36, 44 gleiten. Durch die Abschrägungen 22, 40 an den Stirnseiten 20, 46 der Laschen 18, 38 können geringfügige Abweichungen in der Ausrichtung des Aufsatzes 24 ausgeglichen werden, indem beim Aufstecken der Aufsatz in die korrekte Ausrichtung geführt wird. Der Aufsatz 24 gleitet unter entsprechender Krafteinwirkung bis zu Anschlägen 52, 54 des Befestigungsbereichs 12 (Fig. 4). Von Bedeutung für den erzielten Kraftschluß ist, daß sich das gewölbte Halteelement 16 biegefester verhält, als die biegsame Klinge 14. Das Halteelement 16 kann aus Kunststoff bestehen.

### Bezugszeichenliste

- 10: Handhabe
- 12: Befestigungsbereich
- 14: Klinge
- 16: Halteelement
- 17: Träger
- 17a: Aussparung
- 18: Lasche
- 20: Stirnseite
- 22: Abschrägung
- 24: Aufsatz
- 26: Funktionsrichtung
- 30: Aufsetzrichtung
- 32: flächige Erstreckung
- 34: Ausformung
- 36: Ausnehmung
- 38: Lasche
- 40: Abschrägung
- 42: Ausformung
- 44: Ausnehmung
- 46: Stirnseite
- 48: Griffrand
- 50: Griffrand
- 52: Anschlag
- 54: Anschlag

## Patentansprüche

1. Vorrichtung zur abtragenden Behandlung von Hornhaut, mit einer Handhabe (10), einen sich an die Handhabe (10) anschließenden Befestigungsbereich (12) und einem Aufsatz (24), der eine Baugruppe bestehend aus einem Halteelement (16) und einer Klinge (14) ist, wobei das Halteelement (16) im Befestigungsbereich (12) kraftschlüssig festlegbar ist,
**dadurch gekennzeichnet,**
**daß** die Klinge (14) durch das Halteelement (16) und innerhalb diesem während ihrer gesamten Handhabung derart gehalten ist,
**daß** ein Benutzer ein Austauschen der Klinge (14) durch ein Austauschen des Halteelements (16) ausführen kann, wobei das Halteelement (16) Griffbereiche (48, 50) bietet, an denen ein Benutzer zum Austausch des Aufsatzes (24) den Aufsatz (24) greifen kann, und wobei der Aufsatz (24) derart ausgestaltet ist,
**dass** eine Klinge (14) mit zwei gegenüberliegend angeordneten Schneiden verwendbar ist, so daß bei Verschleiß einer Schneide der Klinge (14) durch umgekehrtes Aufsetzen des Aufsatzes (24) die zweite Schneide der Klinge (14) einsetzbar ist.

2. Vorrichtung nach Anspruch 1, **dadurch gekennzeichnet, daß** als Befestigungsbereich (12) ein mit der Handhabe (10) starr verbundener, gewölbter Träger (17) dient.

3. Vorrichtung nach Anspruch 2, **dadurch gekennzeichnet, daß** sowohl Träger (17) wie auch Halteelement (16) parallel zueinander gewölbt sind und zwischen beiden die unter Biegespannung stehende Klinge (14) angeordnet ist.

4. Vorrichtung nach Anspruch 2 oder Anspruch 3, **dadurch gekennzeichnet, daß** der Träger (17) zumindest eine als Befestigungselement für das Halteelement (16) ausgebildete Lasche (18, 38) aufweist.

5. Vorrichtung nach einem der vorhergehenden Ansprüche, **dadurch gekennzeichnet, daß** die Klinge (14) durch zumindest ein im Befestigungsbereich (12) angeordnetes Rastelement fixierbar ist.

6. Vorrichtung nach einem der vorhergehenden Ansprüche, **dadurch gekennzeichnet, daß** die Abmessungen des Halteelements (16) die Abmessungen der Klinge (14) übersteigen.

7. Vorrichtung nach einem der vorhergehenden Ansprüche, **dadurch gekennzeichnet, daß** das Halteelement (16) quer zur Aufsetzrichtung (30) und senkrecht zur flächigen Erstreckung (32) eine bügelförmige Ausformung (34) mit zumindest einer langlochförmigen Ausnehmung (36, 44) aufweist, in welche eine Lasche (18, 38) des Trägers (17) eingreifen kann.

## Claims

1. Device for the abrasive treatment of calloused skin, with a handle (10), and fastening area (12) adjoining the handle (10) and an attachment (24), which is a component comprising a holding element (16) and a blade (14), whereby the holding element (16) can be fixed rigidly in the fastening area (12),
**characterised in that** the blade (14) is held by and within the holding element (16) during the entire period of use in such a way that a user can replace the blade (14) by replacing the holding element (16), whereby the holding element (16) has grip areas (48, 50) at which, in order to replace the attachment (24) a user can grip the attachment (24), and whereby the attachment (24) is designed in such a way that a blade (14) with two cutting edges arranged opposite each other can be used, so that once one cutting edge of the blade (14) is worn, the second cutting edge of the blade (14) can be deployed by fixing the attachment (24) the other way around.

2. Device in accordance with claim 1 **characterised in that** a convex carrier (17), rigidly connected to the handle (10), serves as the fastening area (12).

3. Device in accordance with claim 2 **characterised in that** both the carrier (17) as well as the holding element (16) are curved in parallel to each other and the flexurally stressed blade (14) is arranged between them both.

4. Device in accordance with claim 2 or claim 3 **characterised in that** the carrier (17) has at least one lug (18, 38) designed as a fastening element for the holding element (16).

5. Device in accordance with any one of the preceding claims **characterised in that** the blade (14) can be fixed by at least one snap-in element arranged in the attachment area (12).

6. Device in accordance with any one of the preceding claims **characterised in that** the dimensions of the holding element (16) exceed the dimensions of the blade (14).

7. Device in accordance with any one of the preceding claims, **characterised in that** the holding element (16) has, laterally to the direction of attachment (30) and perpendicularly to the planar extension (32) a bow-shaped moulding (34) with at least one elongated hole recess (36, 44), into which a lug (18, 38) of the carrier (17) can engage

## Revendications

1. Dispositif pour le traitement abrasif de la corne, avec une poignée (10), une zone de fixation (12) dans le prolongement de la poignée (10) et un chapeau (24), formant un ensemble constitué d'un élément de maintien (16) et d'une lame (14), dans lequel l'élément de maintien (16) peut être fixé par blocage dans la région de fixation (12),
**caractérisé en ce que**
la lame (14) est maintenue de telle manière par l'élément de maintien (16) et à l'intérieur de celui-ci pendant toute la manipulation, que l'utilisateur peut effectuer un remplacement de la lame (14) en remplaçant l'élément de maintien (16), l'élément de maintien (16) offrant des zones de préhension (48, 50), par lesquelles un utilisateur peut saisir le chapeau (24) pour remplacer le chapeau (24), et le chapeau (24) étant conçu de manière à pouvoir utiliser une lame (14) avec deux tranchants opposés, de sorte qu'en cas d'usure d'un tranchant de la lame (14), le deuxième tranchant de la lame (14) peut être utilisé en inversant la disposition du chapeau (24).

2. Dispositif selon la revendication 1, **caractérisé en ce qu'**un support voûté (17), relié fixement à la poignée (10), sert de zone de fixation (12).

3. Dispositif selon la revendication 2, **caractérisé en ce qu'**aussi bien le support (17) que l'élément de maintien (16) sont voûtés parallèlement l'un à l'autre, et la lame (14) sous contrainte de flexion est disposée entre les deux.

4. Dispositif selon la revendication 2 ou la revendication 3, **caractérisé en ce que** le support (17) comporte au moins une languette (18, 38) formée comme un élément de fixation pour l'élément de maintien (16).

5. Dispositif selon l'une des revendications précédentes, **caractérisé en ce que** la lame (14) peut être fixée par au moins un élément d'encliquetage disposé dans la zone de fixation (12).

6. Dispositif selon l'une des revendications précédentes, **caractérisé en ce que** les dimensions de l'élément de maintien (16) dépassent les dimensions de la lame (14).

7. Dispositif selon l'une des revendications précédentes, **caractérisé en ce que** l'élément de maintien (16) comporte une formation en forme d'étrier (34), transversalement au sens de pose (30) et verticalement à l'étendue surfacique (32), avec au moins un évidement (36, 44) en forme de trou oblong, dans lequel peut s'engager une languette (18, 38) du support (17).
